(19) Europäisches Patentamt / European Patent Office / Office européen des brevets

(11) **EP 1 548 033 B1**

(12) **FASCICULE DE BREVET EUROPEEN**

(45) Date de publication et mention
de la délivrance du brevet:
**13.07.2011 Bulletin 2011/28**

(51) Int Cl.:
*C08B 30/12* (2006.01)    *C08B 30/18* (2006.01)
*C08B 30/20* (2006.01)    *C08B 35/00* (2006.01)
*A61M 1/28* (2006.01)    *A61K 47/36* (2006.01)

(21) Numéro de dépôt: **04293056.0**

(22) Date de dépôt: **20.12.2004**

(54) **Polymères solubles de Glucose hautement branchés**

Hochverzweigte lösliche Glukosepolymerisate

Higly branched soluble glucose polymers

(84) Etats contractants désignés:
**AT BE BG CH CY CZ DE DK EE ES FI FR GB GR
HU IE IS IT LI LT LU MC NL PL PT RO SE SI SK TR**

(30) Priorité: **19.12.2003 FR 0315085**

(43) Date de publication de la demande:
**29.06.2005 Bulletin 2005/26**

(73) Titulaire: **Roquette Frères
62136 Lestrem (FR)**

(72) Inventeurs:
• **Fuertes, Patrick
59160 Lomme (FR)**

• **Roturier, Jean-Michel
59930 La Chappelle d'Armentieres (FR)**
• **Petitjean, Carole
59520 Marquette Lez Lille (FR)**

(74) Mandataire: **Pöpping, Barbara et al
Cabinet Plasseraud
52 rue de la Victoire
75440 Paris Cedex 09 (FR)**

(56) Documents cités:
**EP-A- 0 574 721    EP-A- 1 369 432
WO-A-00/18893    WO-A-00/66633
WO-A-02/10427    WO-A-03/018639
FR-A- 2 499 588    US-A1- 2003 134 394**

**Description**

L'invention a pour objet des polymères solubles de glucose hautement branchés présentant une faible teneur en sucres réducteurs, un taux de liaisons glucosidiques $\alpha$-1,6 remarquablement élevé, une gamme étroite de hauts poids moléculaires, et un profil de distribution des longueurs de chaînes branchées tout à fait particulier.

L'invention concerne également des polymères solubles de glucose hautement branchés présentant une faible viscosité intrinsèque.

L'invention destine plus particulièrement ces polymères solubles de glucose hautement branchés à des applications alimentaires et surtout médicales.

Par « profil de distribution des longueurs de chaînes branchées » on entend au sens de l'invention la répartition en taille, exprimée en degré de polymérisation (ou DP), des chaînes glucosidiques linéaires $\alpha$-1,4 reliées à d'autres chaînes glucosidiques linéaires $\alpha$-1,4 par des points de branchement $\alpha$-1,6.

L'invention concerne également un procédé de fabrication desdits polymères solubles de glucose hautement branchés.

Les polymères de glucose accessibles industriellement sont classiquement préparés par hydrolyse des amidons naturels ou hybrides et de leurs dérivés.

Les hydrolysats d'amidon standard sont ainsi produits par hydrolyse acide ou enzymatique d'amidon de céréales ou de tubercules. Ils sont en fait un mélange de glucose et de polymères du glucose, de poids moléculaires extrêmement variés.

Ces hydrolysats d'amidon (dextrines, maltodextrines...) produits dans l'industrie (avec un certain DP moyen) consistent en une large distribution de saccharides contenant à la fois des structures linéaires et branchées.

Les hydrolysats d'amidon, et notamment les maltodextrines sont plus particulièrement utilisées comme transporteurs ou agents de charge, agents texturants, supports d'atomisation, agents filmogènes, agents de contrôle de congélation ou agents anti-cristallisants.

Ils peuvent être également utilisés comme agents de remplacement de matières grasses ou pour leur apport nutritionnel.

Au niveau intestinal, les hydrolysats d'amidon sont ainsi digérés par l'$\alpha$-amylase pancréatique qui agit directement sur les chaînes linéaires liées en $\alpha$-1,4.

Cette digestion enzymatique ciblée conduit à réduire la taille desdits hydrolysats d'amidon jusqu'aux dextrines limites, puis un certain nombre d'enzymes liées à la muqueuse intestinale (maltase, sucrase et $\alpha$-dextrinase) continuent à hydrolyser les saccharides linéaires et les saccharides branchés résiduels en unités glucose.

La cinétique de ces différentes digestions enzymatiques est alors directement fonction de la structure des hydrolysats d'amidon.

Par exemple, l'$\alpha$-amylase pancréatique va agir plus facilement sur les hydrolysats d'amidon riches en oligosaccharides linéaires ou présentant des structures branchées à longues chaînes, tandis qu'elle va agir plus difficilement ou pas du tout sur des structures branchées compactes qui présentent majoritairement des chaînes courtes.

Dans l'état de la technique, ces deux types de structures sont généralement employés différemment, en fonction des domaines d'application visés.

Le premier type de structures dérivées de l'amidon, (notamment celui des oligosaccharides de court DP) est utilisé comme source de glucose directement assimilable par l'organisme, notamment dans trois domaines d'application.

Le premier domaine d'application est celui des substrats énergétiques pour sportifs.

En effet, dans le domaine sportif, une boisson, consommée pendant une activité physique qui requiert beaucoup d'effort, se doit d'apporter instantanément l'énergie et l'eau nécessaire pour compenser la perte de fluide par perspiration.

Il résulte qu'une composition équilibrée en hydrates de carbone est essentielle pour obtenir un tel résultat.

Une solution classiquement proposée pour la boisson optimale est de préférer de courts oligosaccharides linéaires de DP 3 à 6 à des structures glucosidiques branchées plus compactes, puisque ces courts oligosaccharides sont absorbés à la fréquence la plus élevée, tout en gardant l'osmolalité à un niveau modéré, empêchant ainsi la perte des fluides et les effets secondaires tels la diarrhée et les crampes.

Le deuxième domaine d'application est celui de l'alimentation parentérale, où des solutions nutritives apportées par voie veineuse sont conçues pour maintenir un patient en bonne santé et pour lui fournir les nutriments quand il ne peut plus être alimenté via son système digestif normal.

On choisit ici également d'administrer des oligosaccharides linéaires avec un DP compris entre 2 et 5, puisque ces saccharides sont hydrolysés par des maltases dans le rein, et que le glucose libéré est alors réabsorbé. C'est ainsi que l'utilisation de courts oligosaccharides linéaires permet d'apporter suffisamment d'énergie dans une solution isotonique, sans surhydrater le patient.

Le troisième domaine d'application est celui de la nutrition entérale, où il est nécessaire de fournir des boissons qui peuvent être ou bien ingérées oralement ou bien administrées par voie tubulaire dans l'estomac ou l'intestin grêle.

Pour ces fluides entéraux, le problème majeur est cependant la diarrhée, due à une trop forte osmolalité.

Le deuxième type de structures dérivées de l'amidon, i.e. celui des hydrolysats d'amidon ou de dérivés d'amidon présentant des structures branchées compactes à courtes chaînes, est utilisé pour ralentir la libération du glucose assimilable et/ou apporter une certaine osmolalité, notamment dans trois domaines d'applications.

Le premier domaine d'application est celui du domaine de la dialyse péritonéale continue et ambulatoire.

Le brevet EP 207.676 enseigne que pour un usage en dialyse, on préfère aux oligosaccharides linéaires de court DP, des hydrolysats d'amidon renfermant des structures branchées formant des solutions limpides et incolores à 10 % dans l'eau, ayant un poids moléculaire (Mw) de $5.10^3$ à $10^6$ daltons et un indice de polymolécularité ou Ip faible.

Cela se traduit par des compositions qui renferment majoritairement des polymères de glucose de haut poids moléculaire compris entre $5.10^3$ et $5.10^5$ daltons, qui ne renferment pas ou très peu de glucose ou d'oligosaccharides de DP inférieur ou égal à 3, et pas ou très peu de polymères de glucose de Mw supérieur à $10^6$ daltons.

On conçoit en effet aisément pour cette application en dialyse péritonéale que les oligosaccharides de court DP et de faible poids moléculaire traversent rapidement la paroi péritonéale et sont ainsi sans intérêt durable pour la création d'un gradient de pression osmotique, et que les polymères de très haut poids moléculaire, dénués de pouvoir osmotique, sont à éviter et même à proscrire puisque potentiellement dangereux s'il leur advenait de précipiter consécutivement à leur rétrogradation.

Dans son brevet EP 667.356, la société Demanderesse proposait un procédé de fabrication, à partir d'amidon waxy, d'un hydrolysat d'amidon complètement soluble dans l'eau et de faible indice de polymolécularité, inférieur à 2,8, ayant un Mw compris entre $5.10^3$ et $1.10^6$ daltons.

Ce procédé consistait à hydrolyser par voie acide un lait d'amidon constitué exclusivement d'amylopectine, puis à compléter cette hydrolyse acide par une hydrolyse enzymatique à l'aide d' α-amylase bactérienne, et de chromatographier l'hydrolysat obtenu sur résines cationiques fortes macroporeuses sous forme alcaline ou alcalino-terreuse.

Il est à noter qu'à l'époque, la société Demanderesse recommandait de n'utiliser que des amidons presque exclusivement composés d'amylopectine et couramment dénommés amidons waxy ou amidons cireux comme matière première dans ledit procédé, les amidons ou fécules contenant une proportion non négligeable d'amylose ne convenant pas.

Le deuxième domaine d'application est celui de la régulation de la digestion ou de l'alimentation des diabétiques.

Il a été en effet proposé dans le brevet US 4.840.807 ou dans la demande de brevet JP 2001/11101 (n° d'enregistrement 11/187.708), d'extraire les seules zones denses en liaisons α-1,6, comme source de glucides à absorption lente, au sens où les liaisons α-1,6 sont plus difficiles à dégrader que les liaisons α-1,4.

Deux familles de produits ont ainsi été développées. La première concerne les dextrines limites préparées par la dégradation des zones à liaisons α-1,4 par une α-amylase seule, et la deuxième famille concerne les dextrines préparées par la dégradation des zones à liaisons α-1,4 par l'action simultanée d'une α-amylase et d'une β-amylase.

Ces dextrines limites obtenues sont alors particulièrement résistantes aux enzymes digestives humaines.

Cependant, ces composés ont l'inconvénient de présenter un poids moléculaire très faible (compris entre 10.000 et 55.000 daltons), ce qui en limite l'usage dans d'autres domaines d'applications.

Le troisième domaine d'application est celui des substituts du plasma sanguin.

La demande de brevet internationale WO 03/18639 recommande en effet de développer, uniquement à partir d'amylopectine, des composés hyperramifiés pour les utiliser dans le traitement chirurgical ou thérapeutique de mammifères ou dans des méthodes diagnostiques.

Selon l'enseignement de cette demande de brevet, et plus particulièrement dans le domaine des substituts de plasma sanguin, ces amylopectines hyperramifiées sont présentées comme devant permettre de résoudre l'inconvénient majeur des premiers substituts de plasma sanguin élaborés - il s'agit des hydroxyéthylamidons ou HEA - i.e. leur imparfaite métabolisation dans l'organisme.

Dans cette demande de brevet, la stabilité relative desdites amylopectines hyperramifiées est évoquée en relation avec leur teneur élevée en liaisons α-1,6.

Cette teneur élevée en liaisons α-1,6 permettrait alors de réduire suffisamment fortement la dégradation de l'amylopectine à l' α-amylase et de produire un polysaccharide dégradable mais possédant encore les propriétés d'un substitut de plasma sanguin idéal, à savoir ses propriétés pharmacocinétiques et son effet volumique.

D'autre part, la possibilité de faire varier la répartition des points de ramification est également envisagée dans cette demande de brevet WO 03/018639 pour contrôler la cinétique de la dégradation de l'amylopectine hyperramifiée dans la direction souhaitée.

Cependant, la préparation de ces amylopectines hyperramifiées souffre encore d'un inconvénient majeur.

En effet, en raison d'un degré de ramification trop élevé (jusqu'à 25 % de liaisons α-1,6) ou d'une trop courte distance entre les points de ramification, l'effet obtenu est diamétralement opposé à celui souhaité car l'attaque par l' α-amylase de ces amylopectines hyperramifiées peut être fortement retardée ou ne plus intervenir du tout.

L'encombrement stérique est tel, dans les régions de la molécule où la densité des points de ramification est élevée, que l'accès à l' α-amylase n'est plus possible.

Dans ces conditions, l'absence de digestibilité enzymatique de ces amylopectines hyperramifiées ne favorise particulièrement pas l'usage de telles structures comme substituts de plasma sanguin (accumulation de produits non dégradés).

De tout ce qui précède, il résulte qu'il y a donc un besoin non satisfait de disposer de polymères de glucose hautement branchés présentant des propriétés structurales remarquables en termes de répartition des longueurs de chaînes branchées et de viscosité intrinsèque et conférant par-là même aux produits qui les contiennent des capacités plus grandes de durée de vie et de digestibilité contrôlée.

Ces propriétés permettraient alors l'usage de ces polymères de glucose hautement branchés dans des domaines d'application aussi variés que l'apport de substrats énergétiques lors d'activités physiques et dans les domaines de la dialyse péritonéale, la nutrition entérale ou parentérale, les substituts de plasma sanguin, la régulation de la digestion et l'alimentation des diabétiques.

La Société Demanderesse a eu le mérite de concilier tous ces objectifs réputés jusqu'alors difficilement conciliables en imaginant et élaborant, au prix de nombreuses recherches, de nouveaux polymères solubles de glucose hautement branchés tout à fait particuliers en ce qui concerne leur distribution des longueurs de chaînes branchées et leur viscosité intrinsèque.

Les polymères solubles de glucose hautement branchés conformes à l'invention, sont des polymères de glucose présentant une teneur en sucres réducteurs inférieure à 1 %, un taux de liaisons glucosidiques $\alpha$-1,6 compris entre 13 et 17 % et un Mw d'une valeur comprise entre $0,9. 10^5$ et $1,5. 10^5$ daltons, caractérisés par le fait que leur profil de distribution des longueurs de chaînes branchées est constitué de 70 à 85 % de DP inférieur à 15, de 10 à 16 % de DP compris entre 15 et 25 et de 8 à 13 % de DP supérieur à 25.

De manière préférentielle, les polymères solubles de glucose hautement branchés conformes à l'invention présentent un coefficient de viscosité intrinsèque « a » selon la relation de MARK HOUWINK SAKURADA inférieur ou égal à 0,1.

La société Demanderesse a déjà décrit dans sa demande de brevet EP 1.369.432 des polymères solubles de glucose hautement branchés ayant une teneur en sucres réducteurs inférieure à 1 %, un taux de liaisons $\alpha$-1,6 compris entre 12 et 30 % et un Mw d'une valeur comprise entre 0,35 et $2.10^5$ daltons.

Cependant, aucun des polymères de glucose décrits et exemplifiés dans ladite demande de brevet ne présente le profil de distribution des longueurs de chaînes branchés et la viscosité intrinsèque des polymères de glucose hautement branchés conformes à l'invention, comme il sera exemplifié ci-après.

La détermination du pouvoir réducteur, du taux de liaisons glucosidiques $\alpha$-1,6 et des masses moléculaires des polymères solubles de glucose hautement branchés conformes à l'invention sont réalisés dans les mêmes conditions que celles décrites dans la demande de brevet EP 1.369.432.

Les polymères solubles de glucose hautement branchés conformes à l'invention présentent alors une teneur en sucres réducteurs inférieure à 1 %, un taux de liaisons glucosidiques $\alpha$-1,6 compris entre 13 et 17 % et un Mw d'une valeur comprise entre $0,9. 10^5$ et $1,5. 10^5$ daltons.

Par rapport aux polymères solubles de glucose hautement branchés de la demande de brevet EP 1.369.432 de la société Demanderesse, les nouveaux polymères de glucose conformes à l'invention présentent des spectres plus étroits de taux de liaisons glucosidique $\alpha$-1,6 et de poids moléculaire.

Mais les polymères solubles de glucose hautement branchés conformes à l'invention sont surtout caractérisés par leur profil de distribution des longueurs de chaînes.

La détermination de la longueur des chaînes branchées des polymères solubles de glucose hautement branchés conformes à l'invention est réalisée en deux étapes.

Une première étape consiste à débrancher lesdits produits (hydrolyse spécifique de la liaison $\alpha$-1,6) à l'aide d'une isoamylase bactérienne, suivie par une deuxième étape d'identification du degré de polymérisation des oligosaccharides libérés par chromatographie d'exclusion stérique (HPSEC) en comparaison avec des pullulans de taille connus.

Cette technique consiste à peser 50 mg de produits à analyser et y ajouter 3,75 ml d'eau. Après agitation de ce mélange, on ajoute 0,5 ml de DiMéthylSulfOxyde (DMSO) et porte à ébullition sous agitation pendant 30 minutes. On abaisse ensuite la température à 45°C et ajoute 0,25 ml de solution tampon d'acétate de sodium 1M (préalablement amené à pH 3,5 avec de l'acide acétique).

On ajoute ensuite 10 µl d'une isoamylase extraite de *Pseudomonas amyloderasoma* commercialisée par la société HAYASHIBARA (à raison de 59.000 U/mg) et laisse agir à 45°C pendant 1 heure. On procède à ce traitement enzymatique successivement deux fois, puis arrête la réaction par ébullition pendant 3 minutes.

Après ajout de 0,5 ml de n-butanol, agitation et conservation à température ambiante sans agitation pendant 1 heure, le milieu réactionnel est ensuite centrifugé à 2600 tr/min pendant 20 minutes, et le surnageant est déminéralisé à l'aide des résines Amberlite 200 et Amberlite IRA-67 commercialisées respectivement par les sociétés FLUKA et SIGMA.

Une dernière agitation et une filtration sur filtre nylon de porosité 0,45 µm sont réalisées avant d'injecter sur colonne HPSEC.

La chromatographie HPSEC est paramétrée de la façon suivante (sur colonne PWXL oligo commercialisée par TSK et sur colonne SB802 + 803 + 804 + 805 commercialisées par SHODEX) :

- Volume d'injection : 200 μl
- Débit : 0,5 ml/min
- Température des colonnes : 40°C
- Eluant : Nitrate de sodium 0,2 M + azide de Na 0,02 %
- Durée d'élution : 180 min

La taille des oligosaccharides libérés est déterminée par leur temps d'élution par rapport au temps d'élution de pullulans de taille connue.

Les polymères de glucose hautement branchés conformes à l'invention présentent alors un profil de distribution des longueurs de chaînes branchées constitué :

- de 70 à 85 % de DP inférieur à 15
- de 10 à 16 % DP compris entre 15 et 25,
- de 8 à 13 % de DP supérieur à 25.

Ce profil de distribution des longueurs des chaînes branchées conduit à obtenir des structures remarquables par leur contenu élevé en chaînes branchées courtes (70 à 85 % de DP inférieur à 15) et son contenu encore relativement important en chaînes branchées de taille moyenne à longue (15 à 30 % de DP supérieur à 15).

De ce fait, les polymères solubles de glucose hautement branchés conformes à l'invention sont riches en chaînes de DP majoritairement courts (plus de 70 % de DP inférieur à 15), ce qui permet d'obtenir une structure ramifiée compacte majoritairement à courtes chaînes, et renferment cependant encore suffisamment de chaînes de taille moyenne à longue (jusqu'à 30 %), que les α-amylases pancréatiques peuvent aisément digérer pour libérer du glucose assimilable par l'organisme.

Les polymères solubles de glucose hautement branchés conformes à l'invention sont également caractérisés par la valeur de leur coefficient de viscosité intrinsèque « a » selon la relation de MARK, HOUWINK et SAKURADA.

La mesure du coefficient de viscosité intrinsèque « a » selon la relation de MARK, HOUWINK et SAKURADA est utilisée par la société Demanderesse pour illustrer le degré de compacité des polymères solubles de glucose hautement branchés conformes à l'invention.

Il est connu de l'homme du métier que la relation empirique de MARK, HOUWINK et SAKURADA qui relie la viscosité intrinsèque $(\eta)$ d'un polymère à sa masse moléculaire moyenne viscosimétrique (ou $M_V$) est donnée par l'équation suivante :

$$\eta = K \times (M_V)^a$$

où « K » et « a » sont des constantes qui dépendent de la nature du polymère étudié, de la nature du solvant et de la température.

Dans les conditions d'analyses selon l'invention, le solvant utilisé par la société Demanderesse est une solution aqueuse de nitrate 0,2 M.

La constante « a » est liée plus particulièrement au volume hydrodynamique moyen occupé par le polymère dans le solvant considéré.

Il est établi dans l'ét at de la technique que pour un polymère en solution, plus la molécule est repliée sur elle-même, plus la valeur de « a » est faible. Inversement, pour une molécule « fortement déployée », plus la valeur de « a » est élevée.

La mesure du coefficient « a » selon la relation de MARK, HOUWINK et SAKURADA est déterminée par calcul, à l'aide de l'équation suivante :

$$Log\ \eta = Log\ K + a\ Log\ (M_V)$$

On trace la courbe Log $\eta$ en fonction de Log $(M_V)$, dont l'ordonnée à l'origine fournit Log K et la pente de la droite est le coefficient « a » .

Les valeurs de viscosité et de masse moléculaire moyenne viscosimétrique des polymères solubles de glucose hautement branchés conformes à l'invention sont déterminées sur un viscosimètre capillaire VISCOTEK (modèle 250) couplé à un réfractomètre R410, après séparation sur colonnes SHODEX SB 802 + 803 + 805.

Les conditions opératoires chromatographiques sont les suivantes :

- Injection : 100 µl
- Débit : 0,5 ml/min
- Température des colonnes : 35°C
- Eluant : Nitrate de sodium 0,2 M et azide de sodium 0,02 %
- Temps d'analyse : 180 min

Les conditions opératoires pour la détection réfractométrique sont :

- Sensibilité R410 : 16 X
- Température du viscosimètre : 35°C

Le marqueur de débit est une solution de glycérine à 5 % dans l'éluant.

La calibration du détecteur est réalisée à l'aide d'un PolyEthylèneOxyde commercialisé par la société VISCOTEK, de poids moléculaire, de concentration et de viscosité intrinsèque connus.

Le retraitement de cette référence permet de calculer la « mass constant » et la « viscosity constant » du viscosimètre.

Le retraitement du pic d'élution du marqueur de débit permet le calcul du temps de référence et des volumes inter-détecteurs.

Les polymères solubles de glucose hautement branchés conformes à l'invention présentent alors un coefficient « a » calculé selon la relation de MARK, HOUWINK et SAKURADA inférieur à 0,1, ce qui traduit un état de compacité élevé, bien supérieur à l'amylopectine (l'amylopectine standard, dans les mêmes conditions de mesure, présente en effet une valeur du coefficient « a » de 0,33).

Les polymères de glucose hautement branchés ainsi obtenus sont donc particulièrement bien adaptés à leur usage dans des domaines d'application où il est nécessaire de disposer de structures compactes et denses, par exemple en dialyse péritonéale ou en alimentation pour diabétiques.

La détermination de la résistance des polymères solubles de glucose hautement branchés conformes à l'invention, aux enzymes impliquées dans la digestion des glucides alimentaires est également un critère essentiel dans le choix d'un ingrédient alimentaire rentrant dans la composition de formulations à l'usage des sportifs ou destinées par exemple à la nutrition entérale et parentérale.

La société Demanderesse avait estimé le pourcentage de libération du glucose par digestion enzymatique des polymères solubles de glucose hautement branchés décrits dans sa demande de brevet EP 1.369.432, à une valeur comprise entre 50 et 70 %.

Cette résistance à l'hydrolyse est nettement supérieure aux maltodextrines classiques et comparable au glycogène.

Comme il sera exemplifié ci-après, les polymères solubles de glucose hautement branchés conformes à l'invention, libèrent au final du glucose dans des proportions similaires à celles décrites dans ledit brevet EP 1.369.432, ce qui les rend toujours aptes à être utilisés par les sportifs ou pour les nutritions entérale et parentérale, mais cette libération du glucose s'effectue de façon beaucoup plus retardée dans le temps, ce qui les destine avantageusement à des domaines d'application nécessitant de réguler la glycémie comme l'alimentation des diabétiques.

Il n'existe pas, à la connaissance de la Société Demanderesse, de polymères solubles de glucose hautement branchés qui possèdent une telle distribution de leurs longueurs de chaînes branchées qui permet de les mettre en oeuvre dans tous les domaines d'application visées par la présente invention.

De manière avantageuse, les polymères de glucose hautement branchés conformes à l'invention peuvent être classés en trois sous familles.

Ces trois sous familles présentent un profil de distribution des longueurs de chaînes branchées qui diffère sur leur contenu en chaînes moyennes, de DP compris entre 15 et 25.

La première sous famille couvre des polymères hautement branchés qui présentent au moins 14 % à au plus 16 % de DP compris entre 15 et 25.

La deuxième sous famille couvre des polymères hautement branchés qui présentent au moins 12 % à au plus 14 % de DP compris entre 15 et 25.

La troisième sous famille couvre des polymères hautement branchés qui présentent au moins 10 % à au plus 12 % de DP compris entre 15 et 25.

Cette variabilité dans la répartition des chaînes de taille moyenne autorise l'utilisation avantageuse de ces sous-familles au sein des applications alimentaires ou médicales où il est nécessaire de faire varier la digestibilité des polymères solubles de glucose hautement branchés utilisés.

Ces trois sous familles présentent en effet toutes les trois une structure ramifiée constituée de chaînes de taille majoritairement courte, mais dont les proportions variables en chaînes moyennes permettent d'en moduler non seulement le degré de compacité, mais également de contrôler la libération du glucose, comme il sera exemplifié ci-après.

Pour préparer les polymères solubles de glucose branchés conformes à l'invention, on réalise la succession des étapes suivantes qui consistent à :

1) préparer une solution aqueuse d'amidon présentant une teneur en amylose d'au moins 30 % en poids, de préférence comprise entre 35 et 80 % en poids,
2) traiter successivement ladite solution avec une enzyme de branchement puis une β-amylase,
3) effectuer un fractionnement de manière à récupérer les fractions de haut poids moléculaire,
4) recueillir les polymères de glucose hautement branchés ainsi obtenus.

La préparation des polymères de glucose hautement branchés conformes à l'invention est réalisée en modifiant les conditions opératoires déjà décrites dans la demande de brevet EP 1.269.432 de la société Demanderesse.

Tout d'abord, contrairement à ce qui été décrit dans la demande de brevet EP 1.269.432, le choix d'une qualité d'amidon particulière revêt une grande importance.

La société Demanderesse a en effet trouvé que seuls les amidons dont le contenu en amylose dépasse 30 % peuvent servir de matière première dans le procédé de l'invention.

Les maltodextrines, les amidons standards (qui ne renferment pas plus de 30 % d'amylose) ou les amidons de type waxy, quelle que soit leur origine botanique, ne conviennent absolument pas pour la fabrication des polymères solubles de glucose hautement branchés conformes à l'invention.

Il est par ailleurs du mérite de la société Demanderesse d'avoir vaincu un préjugé technique qui veut que pour l'obtention de structure ramifiée stable, compacte et présentant une digestibilité contrôlée, il faille partir d'amidons riches en amylopectine (comme l'enseigne la demande de brevet WO 03/018639).

Comme il sera exemplifié ci-après, la société Demanderesse a trouvé qu'au contraire, c'est par le choix d'amidons riches en amylose utilisés comme substrats de départ qu'il est possible d'obtenir les polymères solubles de glucose hautement branchés conformes à l'invention.

C'est également par le choix de la teneur en amylose des amidons utilisés comme substrats de départ qu'il est possible d'obtenir les trois sous-familles définies ci-avant.

La deuxième étape du procédé conforme à l'invention consiste à traiter ladite solution d'amidon avec une enzyme de branchement.

Dans la demande brevet EP 1.369.432, la société Demanderesse recommandait d'utiliser de 50.000 à 500.000 U d'enzyme de branchement purifiée pour 100 g sec d'amidon ou de dérivé d'amidon, à une température comprise entre 25 et 95°C, de préférence à une température comprise entre 70 et 95°C, pendant une durée de 18 à 24 heures.

Par enzymes de branchement, on entend les enzymes de branchement choisies dans le groupe constitué par les enzymes de branchement du glycogène, les enzymes de branchement de l'amidon et les mélanges quelconques de ces enzymes.

Pour l'obtention des nouveaux polymères de glucose hautement branchés conformes à l'invention, la société Demanderesse recommande de traiter préférentiellement la solution d'amidon riche en amylose avec de 40.000 à 150.000 U d'enzyme de branchement pour 100 g d'amidon, à une température comprise entre 25 et 80°C pendant une durée de 7 à 24 heures, de préférence entre 18 et 24 heures.

La troisième étape du procédé conforme à l'invention consiste à faire agir une β-amylase sur la solution d'amidon ainsi traitée.

Les conditions d'action (température et pH) de cette enzyme sont de faire agir de 0,05 à 0,5 ml de β-amylase de type SPEZYME BBA de GENENCOR (à 1500 DP°/ml) pour 100 g d'amidon à une température de 60°C, un pH de 4,9 à 5, pendant 1 à 3 heures, de préférence pendant 2 heures.

L'unité DP° signifie « Degrees of Diastatic Power », i.e. la quantité d'enzyme contenue dans 0.1 ml d'une solution à 5 % de la préparation d'enzyme qui produirait une quantité de sucres réducteurs suffisante pour réduire 5 ml de liqueur de Fehling lorsque l'échantillon est incubé avec 100 ml de substrat pendant 1 heure à 20°C.

Contrairement à ce qui est décrit dans la demande de brevet EP 1.369.432, il n'est donc pas utilisé une quelconque enzyme choisie dans le groupe constitué de l' α-amylase, la β-amylase, l'amyloglucosidase et l' α-transglucosidase, mais bien de mettre en oeuvre préférentiellement la β-amylase.

La société Demanderesse a en effet trouvé que c'était par le choix de cette enzyme particulière qu'il est possible d'obtenir aisément les polymères solubles de glucose hautement branchés conformes à l'invention.

Au terme de ce traitement complémentaire, les polymères de glucose hautement branchés solubles sont obtenus en mélange avec leurs produits de dégradation enzymatiques, majoritairement constitués de glucose et de maltose.

La quatrième étape du procédé consiste à effectuer un fractionnement à l'aide d'une technique choisie dans le groupe des séparations sur membrane et des chromatographies, de manière à récupérer les fractions de haut poids moléculaire et les fractions de bas poids moléculaire, comme décrit dans la demande de brevet EP 1.369.432 de la société Demanderesse.

Quelle que soit la méthode mise en oeuvre, les profils obtenus permettent la séparation de la fraction polysaccharidique de haut poids moléculaire correspondant aux polymères de glucose hautement branchés solubles conformes à l'invention, des fractions oligosaccharidiques de bas poids moléculaire, constituées pour l'essentiel de glucose et de maltose.

La dernière étape du procédé conforme à l'invention consiste donc à collecter les fractions de haut poids moléculaire correspondant aux polymères de glucose hautement branchés.

Les produits de haut poids moléculaire peuvent être rassemblés en tant que tels, précipités par de l'éthanol, purifiés et séchés sous vide pendant 24 heures ou encore atomisés, par toute technique connue de l'homme du métier.

Comme il sera exemplifié ci-après, la société Demanderesse a enfin trouvé que pour faire varier le contenu en chaînes moyennes branchées (DP compris entre 15 et 25) des polymères solubles de glucose branchés conformes à l'invention, il faut faire varier le contenu en amylose de l'amidon.

En effet, plus le contenu en amylose de l'amidon utilisé comme substrat de départ est élevé, plus le contenu en chaînes ramifiées de DP compris entre 15 et 25 des produits obtenus est faible.

On choisit préférentiellement un amidon dont le contenu en amylose est compris entre au moins 30 % et au plus 40 % pour obtenir la première famille des polymères conformes à l'invention, présentant au moins 14 % à au plus 16 % de DP compris entre 15 et 25. Les amidons de pois standard conviennent particulièrement bien pour l'obtention de cette première famille.

On choisit préférentiellement un amidon dont le contenu en amylose est compris entre au moins 40 % et au plus 60 % pour obtenir la deuxième famille des polymères conformes à l'invention, présentant au moins 12 % à au plus 14 % de DP compris entre 15 et 25.

On choisit enfin un amidon dont le contenu en amylose est compris entre au moins 60 % et au plus 80 % pour obtenir la deuxième famille des polymères conformes à l'invention, présentant au moins 10 % à au plus 12 % de DP compris entre 15 et 25.

Les caractéristiques physico-chimiques particulières des polymères selon l'invention les destinent avantageusement aux applications alimentaires et médicales et plus particulièrement encore comme apport de substrats énergétiques lors d'activités physiques et dans les domaines de la dialyse péritonéale, la nutrition entérale ou parentérale, les substituts de plasma sanguin, la régulation de la digestion et l'alimentation des diabétiques.

D'autres caractéristiques et avantages de l'invention apparaîtront à la lecture des exemples non limitatifs décrits ci-dessous.

**Exemple 1**

On prépare une solution d'amidon à 10 % de matière sèche à partir d'un amidon de pois présentant une richesse en amidon supérieure à 95 % et une teneur en amylose de 36,7 %.

Pour cela, on remet en suspension 100 g sec d'amidon de pois dans un litre d'eau à température ambiante et sous agitation.

La solubilisation totale de l'amidon est réalisée dans un cuiseur à 145 °C pendant 3 à 4 minutes, puis refroidissement à 70°C. On ajoute en continu l'enzyme de branchement du glycogène purifiée de *Bacillus stearothermophilus* à raison de 1 ml de solution d'enzyme à 50.000 U/ml pour 100 g sec de substrat.

La réaction enzymatique est menée pendant 21 heures à 70°C et à pH 6,8 puis stoppée par chauffage à 90°C pendant 1 h.

Le traitement complémentaire avec 0,15 ml de β-amylase (BBA SPEZYME de GENENCOR à 1500 DP°/ml) pour 100 g sec d'amidon s'effectue dans le milieu réactionnel précédent amené à la température de 60°C et au pH de 4,9 à 5.

L'incubation est réalisée pendant 2 heures, et la réaction est stoppée par chauffage 1 h à 90°C.

Le milieu réactionnel est ensuite ultrafiltré sur membrane avec un seuil de coupure de 9000 daltons (membrane ES209 de PCI), et l'ultrafiltrat est collecté et atomisé.

Le tableau I suivant présente les résultats des caractéristiques physico-chimiques (taux de liaisons $\alpha$-1,6, Mw et teneur en sucres réducteurs) du polymère soluble de glucose branché conforme à l'invention ainsi obtenu.

**Tableau I**

| Taux de liaisons $\alpha$-1,6 (%) | 14 |
|---|---|

(suite)

| | |
|---|---|
| Mw (10$^5$ daltons) | 0,96 |
| Teneur en sucres réducteurs (%) | 0,14 |

Le pourcentage en sucres réducteur est déterminé en suivant la méthode de SOMOGOYI, décrite par N. NELSON dans A photometric adaptation of the SOMOGOYI method for the determination of glucose, 1944, J. Biol. Chem., 153, pp 375-380.

Le profil de distribution des longueurs de chaînes branchées est alors déterminé comme indiqué ci-avant.

Le tableau II suivant présente les résultats obtenus.

**Tableau II**

| | |
|---|---|
| DP 2 à DP 15 (%) | 71,7 |
| DP 15 à DP 25 (%) | 15,4 |
| > DP 25 (%) | 12,9 |

Le polymère soluble de glucose branché obtenu présente une remarquable distribution de ses longueurs de chaînes branchées qui se traduit par un peu plus de 70 % de chaînes courtes (DP inférieur à 15) et un peu moins de 30 % de chaînes moyennes à longues (DP supérieur à 15).

La valeur du paramètre « a » de MARK HOUWINK SAKURADA, déterminé par la méthodologie décrite ci-avant est de 0,1.

Le polymère soluble de glucose branché conforme à l'invention présente donc une structure compacte, repliée sur elle-même, avec cependant encore des chaînes accessibles aux attaques enzymatiques.

**Exemple 2**

On réalise une étude comparative des profils de distribution des longueurs de chaînes branchées des produits de la réaction par enzyme de branchement et β-amylase sur des substrats renfermant des teneurs variables en amylose.

Sont alors traités de la même façon que décrit dans l'exemple 1, de l'amidon de maïs waxy (Amidon A), de l'amidon de maïs standard (amidon B), et deux variétés d'amidon riche en amylose renfermant respectivement 50 et 70 % d'amidon (Amidons D et E). Figurent également dans ce tableau les résultats obtenus avec l'amidon de pois (Amidon C) obtenus dans l'exemple 1.

Le tableau III suivant présente les quantités d'enzymes de branchement (incubation de 18 à 21 heures) et de β-amylase (traitement de 2 heures) utilisées pour le traitement des différentes variétés d'amidon.

**Tableau III**

| Amidons de départ | A | B | C | D | E |
|---|---|---|---|---|---|
| Enzyme de branchement (ml pour 100 g) | 1 | 1 | 1 | 1,5 | 2 |
| SPEZYME BBA (%) | 0,05 | 0,1 | 0,15 | 0,13 | 0,1 |

Le tableau IV présente les résultats obtenus en termes de taux de branchement α-1,6, poids moléculaires et teneurs en sucres réducteurs des polymères de glucose hautement branchés ainsi préparés.

Les composés sont identifiés produits F, G, H, I et J, obtenus respectivement à partir des amidons A, B, C, D et E. Les caractéristiques du produit H sont reprises de l'exemple 1.

**Tableau IV**

| Produits de la réaction | F | G | H | I | J |
|---|---|---|---|---|---|
| Taux de liaisons α-1,6 (%) | 11,2 | 13,3 | 14 | 15,2 | 13,1 |
| Mw (10$^5$ daltons) | 0,84 | 0,77 | 0,96 | 0,96 | 1,31 |
| Teneur en sucres réducteurs (%) | 0,2 | 0,015 | 0,14 | 0,39 | 0,29 |

Les traitements enzymatiques sur les trois substrats riches en amylose (teneurs respectives en amylose de 37,5, 50 et 70 %) permettent de fabriquer des polymères solubles de glucose branchés présentant un taux de branchement $\alpha$-1,6 dans la gamme étroite de 13 à 15 %, pour un poids moléculaire compris entre 0,9 à 1,5. $10^5$ daltons.

Le traitement de l'amidon de maïs waxy et de l'amidon de maïs standard, par ailleurs décrit dans la demande de brevet EP 1.369.432, conduit à des polymères de glucose branchés, présentant un taux de liaisons $\alpha$-1,6 dans la gamme de 11 à 13 % et une valeur de poids moléculaire compris dans la gamme de 0,7 à 0,9 $10^5$ daltons.

L'utilisation des amidons riches en amylose comme substrat de départ permet donc, de façon remarquable, d'obtenir des polymères solubles de glucose hautement branchés présentant des taux de liaisons glucosidiques $\alpha$-1,6 et des poids moléculaires plus élevés que ceux obtenus à partir des substrats plus riches en amylopectine, ce qui est en soi remarquable.

Le tableau V suivant rassemble les différents profils de distribution des longueurs de chaînes des différents produits obtenus. Il y figure également les valeurs du coefficient «de viscosité intrinsèque « a » selon MARK HOUWINK SAKURADA.

**Tableau V**

|  | F | G | H | I | J |
|---|---|---|---|---|---|
| DP 2 à DP 15 | 63,8 | 71,9 | 71,7 | 78,3 | 80,4 |
| DP 15 à DP 25 | 21,8 | 16,3 | 15,4 | 12,6 | 11,5 |
| > DP 25 | 14,4 | 11,8 | 12,9 | 9,1 | 8,1 |
| Coefficient de viscosité intrinsèque « a » | 0,12 | 0,12 | 0,1 | 0,08 | 0,09 |

On constate que les polymères solubles de glucose hautement branchés obtenus à partir d'amidons riches en amylose, présentent de 70 à 85 % de chaînes courtes, de 10 à 16 % de chaînes moyennes et de 8 à 13 % de chaînes longues.

Les polymères solubles de glucose hautement branchés obtenus à partir d'amidons riches en amylopectine contiennent moins de 72 % de chaînes courtes, plus de 16 % de chaînes moyennes et entre 11 et 15 % de chaînes longues.

On constate également que plus la teneur en amylose des amidons traités est importante, plus les polymères solubles de glucose hautement branchés obtenus présentent une composante faible en chaînes de taille moyenne.

Les polymères solubles de glucose hautement branchés conformes à l'invention présentent donc surtout un contenu en chaînes moyennes inférieur à 16 %, et de préférence compris entre 10 et 16 % (ce qui n'est pas le cas des polymères solubles de glucose hautement branchés préparés à partir d'amidon de maïs standard et d'amidon waxy).

Les mesures du coefficient de viscosité intrinsèque « a » selon MARK HOUWINK SAKURADA traduisent également la différence dans le degré de compacité des polymères solubles de glucose hautement branchés obtenus.

On constate que seuls les amidons riches en amylose permettent d'obtenir des polymères solubles de glucose hautement branchés présentant une valeur de coefficient de viscosité intrinsèque inférieur ou égal à 0,1.

Par ailleurs, on constate également qu'il est possible de faire varier le degré de compacité des polymères solubles de glucose branchés conformes à l'invention en faisant varier le contenu en amylose des amidons utilisés comme substrats des réactions enzymatiques.

En conséquence, les structures les plus compactes sont en fait obtenues à partir des amidons les plus riches en amylose.

**Exemple 3**

Pour déterminer le degré de libération du glucose, on prépare des solutions aqueuses de polymères solubles de glucose hautement branchés conformes à l'invention, que l'on met en contact avec une amylase d'origine pancréatique et d'une amyloglucosidase intestinale (poudre acétonique d'intestin).

L'hydrolyse est suivie dans le temps par mesure du glucose apparaissant au cours du temps dans le milieu réactionnel.

Ce test permet d'évaluer la résistance des polymères à l'hydrolyse par les enzymes impliquées dans la digestion des glucides alimentaires.

Deux polymères conformes à l'invention (les produits H et I de l'exemple 2) sont testés en comparaison avec les polymères de glucose obtenus à partir d'amidon standard (le produit G de l'exemple 2) dont l'analyse a été faite dans la demande de brevet EP 1.369.432 de la société Demanderesse, en comparaison avec le glycogène.

Les conditions opératoires pour la digestion enzymatique sont les suivantes :

On pèse précisément 0,6 g de produit à tester.

On ajoute 150 ml de tampon maléate de sodium 0,1 mol/l à pH 7 et agite jusqu'à la dissolution du produit.

On place la solution obtenue au bain-marie pendant 15 minutes, pour que la température de la solution soit de 37°C.

On prélève 1,5 ml de la solution, ajoute 0,15 g de pancréatine de porc et incube pendant 30 min.

On arrête la réaction enzymatique en plaçant les prélèvements dans un bain à sec à 100°C, pendant 10 minutes.

On ajoute 0,75 g de pancréatine de porc et incube pendant 3 h 30 à 37°C sous bain thermostaté et sous agitation.

Des prélèvements sont réalisés régulièrement au cours de l'hydrolyse enzymatique.

On dose alors le glucose des prélèvements pour calculer le pourcentage d'hydrolyse du produit étudié.

Ce dosage est réalisé à l'aide d'une méthode colorimétrique sur un automate HITACHI 704 (ROCHE). Le réactif utilisé est un réactif contenant les enzymes glucose oxydase / peroxydase (GOD/PAP). Le volume de réactif utilisé est de 500 $\mu$l, le volume d'échantillon est de 5 $\mu$l et la température de la réaction est de 30°C.

Les résultats sont présentés dans le tableau VI suivant.

**Tableau VI**

| Glucose libéré : | Produit G | Produit H | Produit I |
|---|---|---|---|
| Entre 30 et 60 minutes (% sur sec) | 36,52 | 27,77 | 29,91 |
| Entre 60 et 120 minutes (% sur sec) | 27,59 | 35,27 | 27,57 |

On constate qu'entre 30 et 60 minutes de réaction, les polymères solubles de glucose hautement branchés conformes à l'invention (produits H et I) préparés respectivement à partir d'amidon standard de pois et d'amidon contenant 50 % d'amylose, libèrent moins rapidement leur glucose que le polymères de glucose (produit G) préparé à partir d'amidon de maïs standard.

Cependant, ce phénomène s'inverse entre 60 et 120 minutes, le produit H libérant plus de glucose que le produit G.

Il apparaît clairement que les deux polymères solubles de glucose hautement branchés H et I conformes à l'invention, préparés à partir d'amidon riche en amylose, peuvent être mis en oeuvre aussi bien en nutrition pour sportifs (le produit H libérant après deux heures de digestion 63 % de glucose avec un effet retard), mais également dans les applications où il est nécessaire de réguler la glycémie (le produit I libérant au cours du temps une plus faible quantité de glucose que les autres produits).

On constate que le choix de la teneur en amylose de l'amidon de départ conditionne l'utilisation des polymères solubles de glucose hautement branchés conformes à l'invention dans des domaines d'application bien définis.

**Revendications**

1. Polymères solubles de glucose hautement branchés, présentant une teneur en sucres réducteurs inférieure à 1 %, un taux de liaisons glucosidiques $\alpha$-1,6 compris entre 13 et 17 % et un Mw, d'une valeur comprise entre $0,9 \cdot 10^5$ et $1,5 \cdot 10^5$ daltons, **caractérisés par le fait que** leur profil de distribution des longueurs de chaînes branchées est constitué de 70 à 85 % de DP inférieur à 15, de 10 à 16 % de DP compris entre 15 et 25 et de 8 à 13 % de DP supérieur à 25.

2. Polymères selon la revendication 1, **caractérisés par le fait qu'**ils présentent un coefficient de viscosité intrinsèque « a » selon la relation de MARK HOUWINK SAKURADA inférieur ou égal à 0,1.

3. Polymères selon l'une ou l'autre des revendications 1 et 2, **caractérisés par le fait qu'**ils présentent entre au moins 14 % et au plus 16 % de DP compris entre 15 et 25.

4. Polymères selon l'une ou l'autre des revendications 1 et 2, **caractérisés par le fait qu'**ils présentent entre au moins 12 % et au plus 14 % de DP compris entre 15 et 25.

5. Polymères selon l'une ou l'autre des revendications 1 et 2, **caractérisés par le fait qu'**ils présentent entre au moins 10 % et au plus 12 % de DP compris entre 15 et 25.

6. Procédé de préparation des polymères solubles de glucose hautement branchés selon l'une quelconque des revendications 1 à 5, **caractérisé par le fait que** l'on :

1) prépare une solution aqueuse d'amidon présentant une teneur en amylose d'au moins 30 % en poids, de préférence comprise entre 35 et 80 % en poids,
2) traite successivement ladite solution avec une enzyme de branchement puis une β-amylase,
3) effectue un fractionnement de manière à récupérer les fractions de haut poids moléculaire,
4) recueille les polymères de glucose hautement branchés ainsi obtenus.

**7.** Procédé de préparation des polymères solubles de glucose hautement branchés selon la revendication 6, **caractérisé par le fait que** l'on traite la solution aqueuse d'amidon :

- avec de 40.000 à 150.000 U d'enzyme de branchement pour 100 g d'amidon, à une température comprise entre 25 et 80°C pendant une durée de 7 à 24 heures, de préférence entre 18 et 24 heures,
- puis avec de 0,05 à 0,5 % ml de β-amylase pour 100 g d'amidon, à une température de 60°C, un pH de 4,9 à 5, pendant une durée comprise entre 1 et 3 heures, de préférence pendant 2 heures.

**8.** Procédé selon l'une ou l'autre des revendications 6 et 7 susceptible de produire les polymères solubles de glucose hautement branchés de la revendication 3, **caractérisé en ce que** la solution aqueuse d'amidon présente entre au moins 30 % et au plus 40 % en poids d'amylose, et est préférentiellement de l'amidon de pois standard.

**9.** Procédé selon l'une ou l'autre des revendications 6 et 7 susceptible de produire les polymères solubles de glucose hautement branchés de la revendication 4, **caractérisé en ce que** la solution aqueuse d'amidon présente entre au moins 40 % et au plus 60 % en poids d'amylose.

**10.** Procédé selon l'une ou l'autre des revendications 6 et 7 susceptible de produire les polymères solubles de glucose hautement branchés de la revendication 5, **caractérisé en ce que** la solution aqueuse d'amidon présente entre au moins 60 % et au plus 80 % en poids d'amylose standard.

**11.** Utilisation des polymères de glucose hautement branchés selon l'une quelconque des revendications 1 à 5 ou susceptibles d'être obtenus par le procédé des revendications 6 à 10 dans des applications alimentaires et médicales.

**12.** Utilisation selon la revendication 11 comme apport de substrats énergétiques lors d'activités physiques et dans les domaines de la dialyse péritonéale, la nutrition entérale ou parentérale, les substituts de plasma sanguin, la régulation de la digestion et l'alimentation des diabétiques.

**Claims**

**1.** Soluble highly branched glucose polymers, having a reducing sugar content of less than 1%, a level of $\alpha$-1,6 glucoside bonds of between 13 and 17% and a Mw of a value of between $0.9 \cdot 10^5$ and $1.5 \cdot 10^5$ daltons, **characterised in that** their branched chain length distribution profile consists of 70 to 85% of DP of less than 15, 10 to 16% of DP of between 15 and 25 and 8 to 13% of DP greater than 25.

**2.** Polymers according to claim 1 **characterised in that** they have an intrinsic coefficient of viscosity "a" according to the MARK, HOUWINK and SAKURADA equation of less than or equal to 0.1.

**3.** Polymers according to either one of claims 1 and 2 **characterised in that** they have between at least 14% and at most 16% of DP of between 15 and 25.

**4.** Polymers according to either one of claims 1 and 2 **characterised in that** they have between at least 12% and at most 14% of DP of between 15 and 25.

**5.** Polymers according to either one of claims 1 and 2 **characterised in that** they have between at least 10% and at most 12% of DP of between 15 and 25.

**6.** A process for preparing the soluble highly branched glucose polymers according to any one of claims 1 to 5, **characterised by**:

1) preparing an aqueous starch solution having an amylose content of at least 30% by weight, preferably between 35 and 80% by weight,

2) successively treating said solution with a branching enzyme and then a β-amylase,
3) carrying out a fractionation step so as to recover the high molecular weight fractions,
4) collecting the highly branched glucose polymers thus obtained.

**7.** A process for preparing the soluble highly branched glucose polymers according to claim 6 **characterised in that** the aqueous starch solution is treated:

- with 40 000 to 150 000 U of branching enzyme per 100 g of starch at a temperature of between 25 and 80°C for a period of 7 to 24 hours, preferably between 18 and 24 hours,
- and then with 0.05 to 0.5% ml of β-amylase per 100 g of starch at a temperature of 60°C, at a pH of 4.9 to 5, for a period of between 1 and 3 hours, preferably for 2 hours.

**8.** A process according to either one of claims 6 and 7 capable of producing the soluble highly branched glucose polymers of claim 3, **characterised in that** the aqueous starch solution has between at least 30% and at most 40% by weight of amylose, and is preferably standard pea starch.

**9.** A process according to either one of claims 6 and 7 capable of producing the soluble highly branched glucose polymers of claim 4, **characterised in that** the aqueous starch solution has between at least 40% and at most 60% by weight of amylose.

**10.** A process according to either one of claims 6 and 7 capable of producing the soluble highly branched glucose polymers of claim 5, **characterised in that** the aqueous starch solution has between at least 60% and at most 80% by weight of standard amylose.

**11.** Use of the highly branched glucose polymers according to any one of claims 1 to 5 or capable of being produced by the process of claims 6 to 10 in food and medical applications.

**12.** Use according to claim 11 as a source of high-energy substrates during physical activities and in the fields of peritoneal dialysis, enteral or parenteral nutrition, blood plasma substitutes, digestion regulation and food for diabetics.

**Patentansprüche**

**1.** Hochverzweigte lösliche Glucosepolymere, die einen Gehalt an reduzierenden Zuckern von weniger als 1%, einen Grad von $\alpha$ -1,6-glykosidischen Bindungen zwischen 13 und 17% und ein Mw mit einem Wert zwischen 0,9. $10^5$ und 1,5. $10^5$ Dalton aufweisen, **dadurch gekennzeichnet, dass** ihr Verteilungsprofil der Längen der verzweigten Ketten aus von 70 bis 85% eines DP von kleiner als 15, von 10 bis 16% eines DP zwischen 15 und 25 und von 8 bis 13% eines DP von größer als 25 besteht.

**2.** Polymere gemäß Anspruch 1, **dadurch gekennzeichnet, dass** sie einen intrinsischen Viskositätskoeffizienten "a" gemäß der MARK-HOUWINK-SAKURADA-Gleichung von kleiner als oder gleich 0,1 aufweisen.

**3.** Polymere gemäß einem der Ansprüche 1 und 2, **dadurch gekennzeichnet, dass** sie zwischen mindestens 14% und höchstens 16% eines DP zwischen 15 und 25 aufweisen.

**4.** Polymere gemäß einem der Ansprüche 1 und 2, **dadurch gekennzeichnet, dass** sie zwischen mindestens 12% und höchstens 14% eines DP zwischen 15 und 25 aufweisen.

**5.** Polymere gemäß einem der Ansprüche 1 und 2, **dadurch gekennzeichnet, dass** sie zwischen mindestens 10% und höchstens 12% eines DP zwischen 15 und 25 aufweisen.

**6.** Verfahren zur Herstellung von hochverzweigten löslichen Glucosepolymeren gemäß einem der Ansprüche 1 bis 5, **dadurch gekennzeichnet, dass**:

1) eine wässrige Stärkelösung hergestellt wird, die einen Amylosegehalt von mindestens 30 Gewichts-%, bevorzugt von zwischen 35 und 80 Gewichts-%, aufweist,
2) die Lösung nacheinander mit einem Verzweigungsenzym, danach mit einer β -Amylase behandelt wird,

3) eine Fraktionierung durchgeführt wird, um die Fraktionen mit hohem Molekulargewicht zu gewinnen,
4) die so erhaltenen hochverzweigten Glucosepolymere gesammelt werden.

7. Verfahren zur Herstellung von hochverzweigten löslichen Glucosepolymeren gemäß Anspruch 6, **dadurch gekennzeichnet, dass** die wässrige Stärkelösung behandelt wird:

- mit von 40.000 bis 150.000 U an Verzweigungsenzym pro 100 g an Stärke, bei einer Temperatur zwischen 25 und 80°C für eine Dauer von 7 bis 24 Stunden, bevorzugt zwischen 18 und 24 Stunden,
- danach mit von 0,05 bis 0,5% ml an β-Amylase pro 100 g an Stärke, bei einer Temperatur von 60°C, einem pH-Wert von 4,9 bis 5, für eine Dauer von zwischen 1 und 3 Stunden, bevorzugt für 2 Stunden.

8. Verfahren gemäß einem der Ansprüche 6 und 7, das dazu geeignet ist, die hochverzweigten löslichen Glucosepolymere nach Anspruch 3 herzustellen, **dadurch gekennzeichnet, dass** die wässrige Stärkelösung zwischen mindestens 30 Gewichts-% und höchstens 40 Gewichts-% Amylose aufweist, und bevorzugt Standard-Erbsenstärke ist.

9. Verfahren gemäß einem der Ansprüche 6 und 7, das dazu geeignet ist, die hochverzweigten löslichen Glucosepolymere nach Anspruch 4 herzustellen, **dadurch gekennzeichnet, dass** die wässrige Stärkelösung zwischen mindestens 40 Gewichts-% und höchstens 60 Gewichts-% Amylose aufweist.

10. Verfahren gemäß einem der Ansprüche 6 und 7, das dazu geeignet ist, die hochverzweigten löslichen Glucosepolymere nach Anspruch 5 herzustellen, **dadurch gekennzeichnet, dass** die wässrige Stärkelösung zwischen mindestens 60 Gewichts-% und höchstens 80 Gewichts-% Standard-Amylose aufweist.

11. Verwendung von hochverzweigten Glucosepolymeren gemäß einem der Ansprüche 1 bis 5 oder erhältlich durch das Verfahren der Ansprüche 6 bis 10 in Nahrungsmittel- und medizinischen Anwendungen.

12. Verwendung gemäß Anspruch 11 als Zufuhr von Energiesubstraten bei körperlichen Aktivitäten und auf den Gebieten der Peritonealdialyse, der enteralen oder parenteralen Ernährung, der Blutplasmaersatzmittel, der Verdauungsregulation und der Ernährung von Diabetikern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 207676 A
- EP 667356 A
- US 4840807 A
- JP 2001011101 A
- WO 0318639 A
- WO 03018639 A
- EP 1369432 A
- EP 1269432 A

**Littérature non-brevet citée dans la description**

- **N. NELSON.** A photometric adaptation of the SOMOGOYI method for the determination of glucose,. *J. Biol. Chem.,* 1944, vol. 153, 375-380